# EUROPÄISCHE PATENTANMELDUNG

(11) **EP 1 997 825 A1**
(43) Veröffentlichungstag der Anmeldung: **03.12.2008**
(21) Anmeldenummer: 07090102.0
(22) Anmeldetag: 24.05.2007
(51) Int. Cl.: C07H 15/04

(54) **Verfahren zur Herstellung von Glycosamin-Analoga**

(71) Anmelder: Universität Potsdam, 14469 Potsdam (DE)
(72) Erfinder: Linker, Torsten, 14469 Potsdam (DE); Elamparuthi, Elangovan, 14469 Potsdam (DE)
(74) Vertreter: Schubert, Klemens

(57) **Zusammenfassung**

Die vorliegende Erfindung betrifft eine einfache Synthese von Kohlenhydrat-2-C-N-Analoga beziehungsweise Glycosamin-Analoga ausgehend von natürlichen Kohlenhydraten beziehungsweise Glycalen.

## Beschreibung

Die vorliegende Erfindung betrifft eine einfache Synthese von Kohlenhydrat-2-C-N-Analoga beziehungsweise Glycosamin-Analoga ausgehend von natürlichen Kohlenhydraten beziehungsweise Glycalen.

Der Einbau von Kohlenstoffresten in Kohlenhydrate und die Synthese von C-Glycosiden ist ein wichtiges Arbeitsgebiet der Organischen Chemie, da Kohlenhydrat-Analoga in Arzneimitteln vorkommen (C-Glycoside Synthesis, M. H. D. Postema, CRC Press, Boca Raton 1995; The Chemistry of C-Glycosides, D. E. Levy, C. Tang, Pergamon 1995; A. Dondoni, A. Marra, Chem. Rev.2000, 100, 4395; L. Somsäk, Chem. Rev.2001, 101, 81).

Bekannt sind auch Arbeiten für die radikalische Addition von Malonsäurediestern an Glycale (ungesättigte Kohlenhydrate) (T. Linker, K. Hartmann, T. Sommermann, D. Scheutzow, E. Ruckdeschel, Angew. Chem.1996, 108, 1819; T. Linker, T. Sommermann, F. Kahlenberg, J. Am. Chem. Soc.1997, 119, 9377; V. Gyóllai, D. Schanzenbach, L. Somsäk, T. Linker, Chem. Commun. 2002, 1294; T. Linker, J. Organomet. Chem.2002, 661, 159.

Allerdings erlaubte diese Methode bisher nur die Einführung von Säure und Estergruppen in die 2-Position von Kohlenhydraten.

Die bekannten Synthesen von Kohlenhydrat-Analoga haben aber den Nachteil, dass sie viele Reaktionsschritte erfordern, sodass die Endprodukte nur in geringen Mengen erhalten werden.

Aufgabe der Erfindung war es daher, ein vereinfachtes Verfahren zur Herstellung von Kohlenhydrat-Analoga zur Verfügung zu stellen.

Die Aufgabe wird gelöst durch ein Verfahren zur Herstellung von Glycosamin-Analoga aus natürlichen Kohlenhydraten, wobei man in einer ersten Stufe
a) das Glycal durch Addition von Nitroalkan zu Kohlenhydrat 2-C-N-Analoga umsetzt, in denen der Stickstoff als Nitrogruppe vorliegt, und dann in einer zweiten Stufe
b) mittels Reduktion Kohlenhydrat C-N-Analoga als Homologe von Glycosaminen erhält.

Bevorzugt ist es, dass die natürlichen Kohlenhydrate ungesättigt sind. Besonders bevorzugt ist es, dass man Fünfringglycale oder Sechsringglycale einsetzt.

Bevorzugt ist ein erfindungsgemäßes Verfahren, bei dem das Nitroalkan 1 bis 4 C-Atome enthält. Die Kohlenstoffkette kann dabei gegebenenfalls auch verzweigt sein.

Besonders bevorzugt ist ein erfindungsgemäßes Verfahren, bei dem man als Glycal ein Sechsringglycal und als Nitroalkan Nitromethan einsetzt.

Besonders bevorzugt ist auch ein erfindungsgemäßes Verfahren, bei dem man als Glycal ein Fünfringglycal und als Nitroalkan Nitroethan einsetzt.

Bevorzugt ist es auch, dass die Reduktion in Schritt b) eine selektive Reduktion ist.

Bevorzugt ist ferner ein erfindungsgemäßes Verfahren, wobei die OH-Gruppen des Glycals geschützt sind. Die Herstellung der Schutzgruppen ist an sich bekannt und beispielsweise beschrieben in "Protective Groups in Organic Synthesis", Peter G. M. Wuts, Theodora W. Greene; Wiley & Sons (2006).

Bevorzugt ist auch ein erfindungsgemäßes Verfahren, wobei die Schutzgruppen der OH-Gruppen im Schritt b) gleichzeitig mit der Bildung des Glycosamins abgespalten werden.

Gleichfalls bevorzugt ist aber auch ein erfindungsgemäßes Verfahren bei dem die Schutzgruppen der OH-Gruppen im Schritt b) bei der Bildung des Glycosamins erhalten bleiben.

Mit der vorliegenden Erfindung können Kohlenhydrat-2-C-N-Analoga ausgehend von einfachen Vorläufern in wenigen Schritten und guten Ausbeuten herstellt werden.

Bei der erfindungsgemäßen Synthese werden Homologe von Glycosaminen in einer zweistufigen Synthese ausgehend von Glycalen hergestellt. Bei Glycalen handelt es sich um Derivate von Zuckermolekülen, die zwischen den Kohlenstoffatomen 1 und 2 eine Doppelbindung besitzen. Glycale sind kommerziell erhältlich oder einfach in einer "Eintopfreaktion" zu synthetisieren.

Interessant ist dabei der Einbau von Stickstoff in Kohlenhydrate, da so Kohlenhydrat-C-Analoga von Glycosaminen erhalten werden können.

Dies gelang erstmals mit der vorliegenden Erfindung, da durch Veränderung der Schutzgruppen auch Radikalreaktionen unter basischen Reaktionsbedingungen möglich wurden. Ausgehend von Glycalen **1**, die kommerziell erhältlich oder im Multigramm-Maßstab zugänglich sind (L. Somsäk, Chem. Rev. 2001, 101, 81; Carbohydrate Builing Blocks, M. Bols, Wiley, New York 1996), wird Nitromethan in Gegenwart von Cer(IV)-ammoniumnitrat (CAN) oder anderen Oxidationsmitteln und Kaliumhydroxid oder anderen Basen addiert, wobei die Kohlenhydrat-2-C-N-Analoga **2** in einem Schritt in guten Ausbeuten synthetisiert werden können. Die Stereoselektivität **(2a : 2b)** ist bei allen Reaktionen sehr gut, die Produkte werden in analysenreiner Form isoliert.

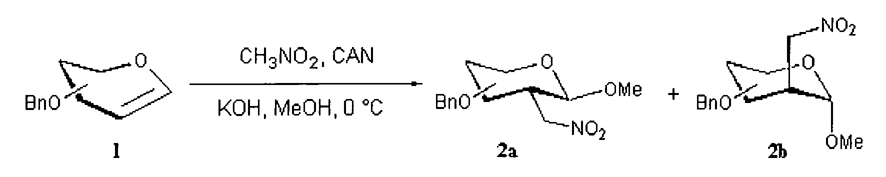

Der Vorteil des Verfahrens besteht in den kostengünstigen Reagenzien und der einstufigen Synthese. Ähnliche Strukturen wie **2** wurden in der Literatur erst einmal beschrieben (K. Koppert, R. Brossmer, Tetrahedron Lett. 1992, 33, 8031.), wobei die Synthese dort ausgehend von Glucose mehr als fünf Reaktionsschritte erforderte. Schließlich konnte in der vorliegenden Erfindung die Reduktion der Nitrogruppe sowohl unter Erhalt der Schutzgruppen zu den Produkten **3** als auch durch katalytische Hydrierung oder andere Reduktionsmittel unter Abspaltung der Schutzgruppen zu den Kohlenhydrat-C-N-Analoga **4** realisiert werden. Bei den Endprodukten handelt es sich um Homologe von Glycosaminen, die ausgehend von den natürlichen Kohlenhydraten mit dem Verfahren in wenigen Stufen synthetisiert werden konnten. Die Verbindungen könnten als Enzyminhibitoren oder Edukte für die Synthese von C-Disaccariden Verwendung finden.

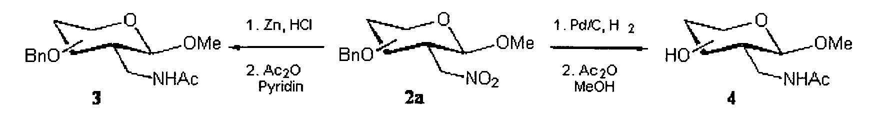

Die durch das erfindungsgemäße Verfahren hergestellten Kohlenhydratanaloga könnten als so genannte Mimetika eingesetzt werden.

Mimetika sind Stoffe, die an bestimmte Rezeptoren binden und somit den Platz des eigentlichen Wirkstoffes belegen. Die Verwendung von Mimetika ist ein erfolgreiches Konzept in der Wirkstoffforschung. Ein prominentes Beispiel eines Kohlenhydratanalogons ist Acarbose, das unter dem Handelsnamen Glucobay^{®} vermarktet wird. Es handelt sich um einen Arzneistoff zur Behandlung der Zuckerkrankheit *(Diabetes mellitus)* Die Wirkung beruht darauf, dass ein Enzym im Darm gehemmt wird, das die Abspaltung von Glucose bewirkt.

Für die Synthese von Kohlenhydratanaloga gibt es viele Strategien und Ansätze ("Carbohydrate Mimics Concepts and Methods" von Yves Chapleur, erschienen bei: Wiley-VCH). Die verschiedenen Zielmoleküle lassen sich auf unterschiedlichen Synthesewegen herstellen. Das erfindungsgemäße Verfahren bietet die Möglichkeit mit wenigen Reaktionsschritten ausgehend von Glycalen Kohlenhydrat-2-C-N-Analoga zu synthetisieren. Diese Synthese in zwei Schritten ausgehend von Glycalen - die käuflich oder leicht herzustellen sind - kann den Weg zu einem gegebnen Zielmolekül erleichtern. Ein weiterer Vorteil sind die Stereoselektivität sowie der Einsatz von günstigen Ausgangsstoffen wie Nitromethan.

In der chemischen beziehungsweise der Pharmaindustrie werden für neue Synthesen alle Möglichkeiten ausgeschöpft, um schnell und entsprechend preiswert zu neuen Produkten zu kommen, dementsprechend groß ist die Aufgeschlossenheit gegenüber neuen Syntheseschritten.

Durch die Erfindung wird eine deutliche Verbesserung in einzelnen Nutzenkategorien erzielt.

Die nachfolgenden Beispiele erläutern die Erfindung.

### Beispiele:

### Allgemeine Vorschrift zur Herstellung der Nitrozucker

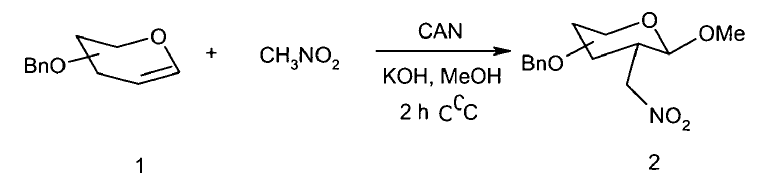

Eine Lösung von Glycal **1** (5,0 mmol), Kaliumhydroxid (560 mg, 10,0 mmol) und Nitromethan (3,05 g, 50 mmol) in Methanol (25 mL) wird auf 0 °C gekühlt. Bei dieser Temperatur tropft man innerhalb von 3 h eine Lösung von getrocknetem Cer(IV)-ammoniumnitrat (10,96 g, 20 mmol) in Methanol (50 mL) zu, bis DC-Kontrolle vollständigen Umsatz zeigt. Nach 30 min bei 0 °C wird eine eiskalte verdünnte Lösung von Natriumbisulfat (200 mL) und Ammoniumchlorid (800 mL) zugegeben und die Mischung mit Dichlormethan extrahiert (3 x 500 mL). Die vereinigten organischen Phasen werden über Natriumsulfat getrocknet, das Lösungsmittel im Vakuum entfernt und das Rohprodukt an Kieselgel (Cyclohexan / Essigester 6 : 1) getrennt. Es werden die Nitroverbindungen **2** in den in Tabelle 1 gegebenen Ausbeuten in analysenreiner Form isoliert.

In analoger Weise lassen sich die Nitrozucker der Fünfringglycale gemäß der folgenden Reaktionsgleichung darstellen:

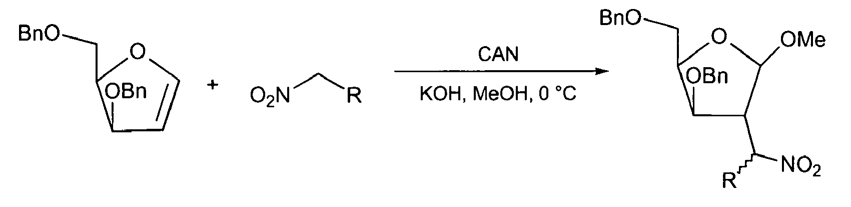

**Tabelle 1**

| Ausgangsverbindung | Produkt | Ausbeute |
|---|---|---|
| (1) | (2) | |
| 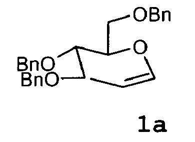 | 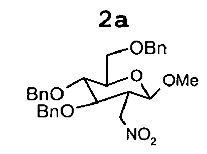 | 59 % |
| 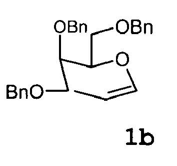 | 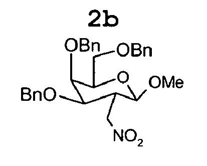 | 55 % |
| 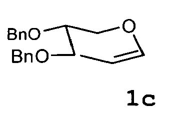 | 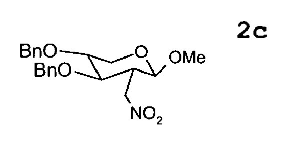 | 28 % |
| 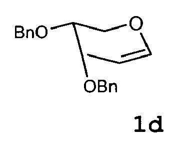 | 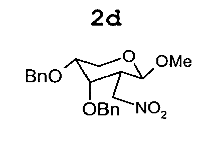 | 31 % |
| 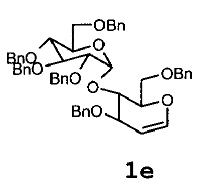 | 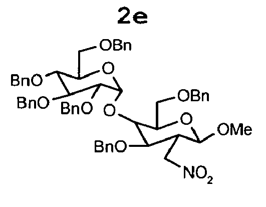 | 58 % |
| 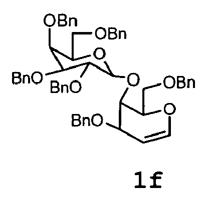 | 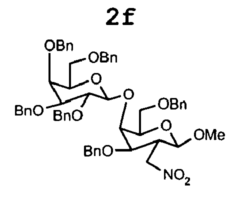 | 59 % |

### Allgemeine Vorschrift zur Herstellung der freien Kohlenhydrate unter Erhalt der Benzylgruppen

### Variante 1

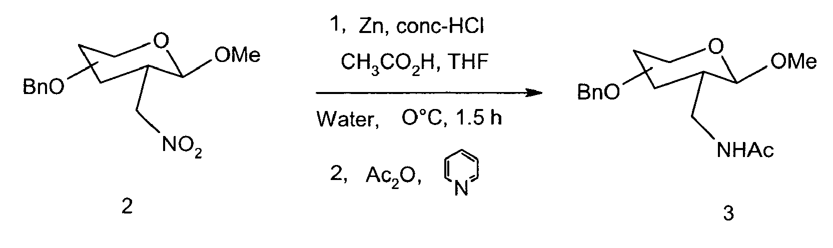

Die Nitroverbindung **2** (1 mmol) wird in einer Mischung aus THF (75 mL), konz. HCl (3 mL), AcOH (16 mL) und Wasser (30 mL) gelöst und die Lösung auf 0 °C gekühlt, Zinkstaub (25 eq) wurde bei dieser Temperatur hinzugefügt. Nach 1 stündigem Rühren bei 0 °C wurde die Vollständigkeit der Reaktion mit Dünnschichtchromatographie überwacht. Zinkstaub wurde durch Filtration über Celite und das Filtrat mit Dichlormethan (50 mL) verdünnt, mit Wasser und gesättigter NaHCO₃ -Lösung gewaschen, um überschüssige Säure zu entfernen und über wasserfreiem Na₂SO₄ getrocknet. Nach dem Verdampfen der Lösemittel wurde das freie Amin in einer Mischung aus Pyridin (2 mL) and Acetanhydrid (1 mL) gelöst und das acetylierte Produkt wurde mittels Säulenchromatographie gereinigt (Kieselgel; Hexan : Ethylacetat 2:1). Es werden die acetylierten Verbindungen **3** in den in Tabelle 2 angegebenen Ausbeuten erhalten.

**Tabelle 2**

| Nitrozucker | Acetyliertes Amin | Ausbeute |
|---|---|---|
| (2) | (3) | |
| 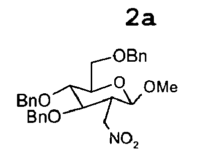 | 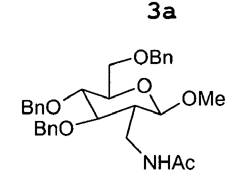 | 81 % |
| 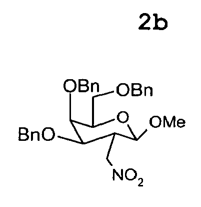 | 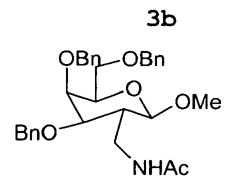 | 79 % |
| 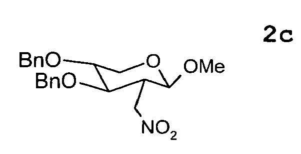 | 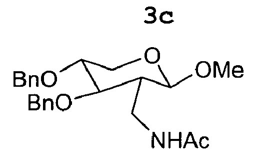 | 69 % |
| 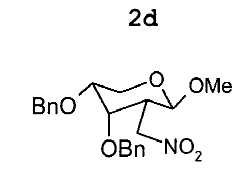 | 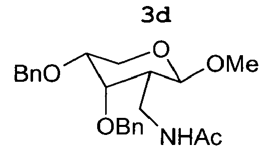 | 75 % |
| 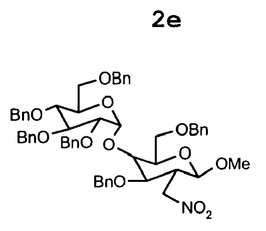 | 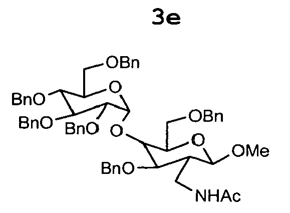 | 71 % |
| 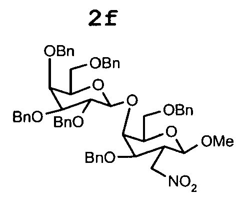 | 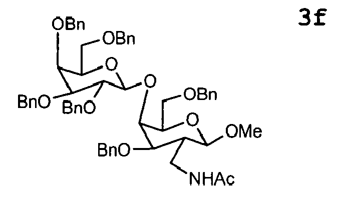 | 74 % |

### Allgemeine Vorschrift zur Herstellung der freien Kohlenhydrate unter Erhalt der Benzylgruppen

### Variante 2

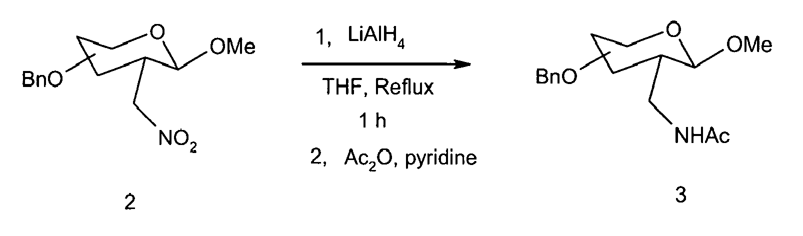

Zu einer Lösung der Nitroverbindung **2** (1 mmol) in THF (5 mL) wurde Lithiumaluminumhydrid (2 eq) hinzugefügt. Die Reaktionsmischung wurde erhitzt, bis Dünnschichtchromatographie die vollständige Umsetzung anzeigte. Die Mischung wurde mit Wasser versetzt, mit Ethylacetat extrahiert und über wasserfreiem Na₂SO₄ getrocknet. Nach dem Verdampfen des Lösemittels wurde das freie Amin ein einer Mischung aus Pyridin (2 mL) and Acetanhydrid (1 mL) gelöst und das acetylierte Produkt wurde mittels Säulenchromatographie gereinigt (Kieselgel; Hexan : Ethylacetat 2:1). Es werden die acetylierten Verbindungen **3** in den in Tabelle 3 angegebenen Ausbeuten erhalten.

**Tabelle 3**

| Nitrozucker | Acetyliertes Amin | Ausbeute |
|---|---|---|
| (2) | (3) | |
| 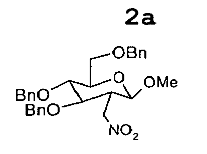 | 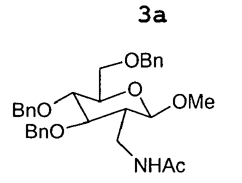 | 83 % |
| 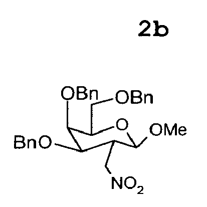 | 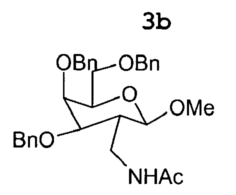 | 81 % |
| 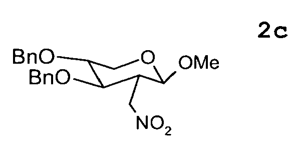 | 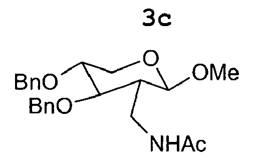 | 78 % |
| 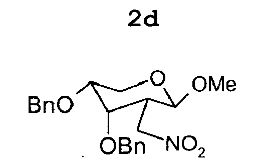 | 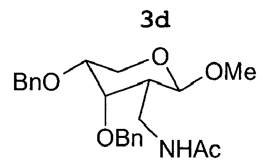 | 82 % |
| 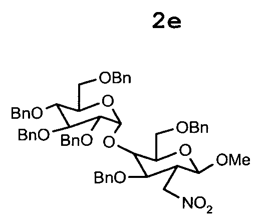 | 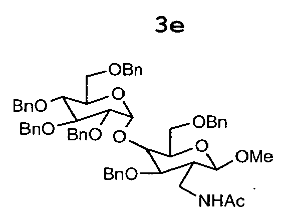 | 79 % |
| 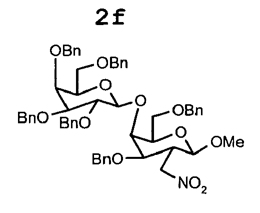 | 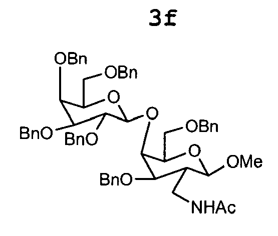 | 80 % |

### Allgemeine Vorschrift zur Herstellung der freien Kohlenhydrate unter Abspaltung der Benzylgruppen

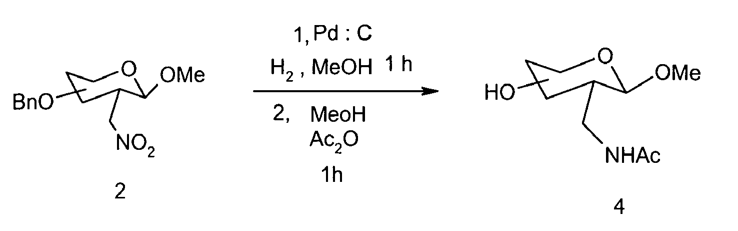

Eine Lösung der Nitroverbindung **2** (1 mmol) in Methanol (5 mL) wird mit Palladium auf Kohle (60 mg) versetzt. In einem Autoklaven wird bei 40 bar mit Wasserstoff hydriert, bis DC-Kontrolle vollständigen Umsatz anzeigt (14 h). Die Mischung wird durch Celite filtriert, mit Methanol gewaschen (3 x 5 mL) und nach Zugabe von Acetanhydrid (0,5 mL) für 30 min acetyliert. Die Lösungsmittel werden im Vakuum entfernt und der Rückstand an Kieselgel (Essigsäureethylester / Methanol 8 : 2) gereinigt. Es werden die freien Kohlenhydrate **4** in den in Tabelle 4 angegebenen Ausbeuten in analysenreiner Form isoliert.

**Tabelle 4**

| **Nitrozucker** | **Acetyliertes Amin** | **Ausbeute** |
|---|---|---|
| **(2)** | **(4)** | |
| 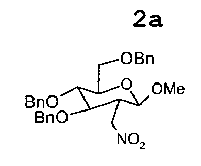 | 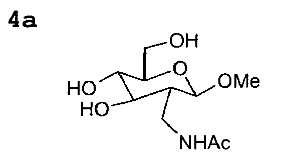 | 82 % |
| 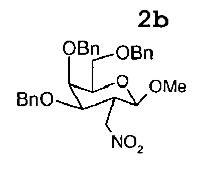 | 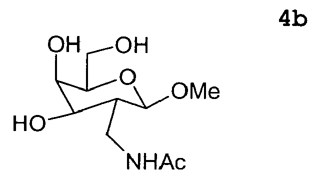 | 77 % |
| 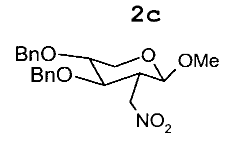 | 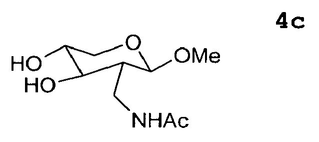 | 78 % |
| 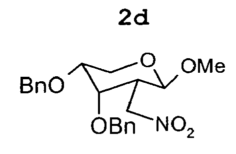 | 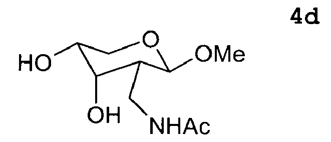 | 75 % |
| 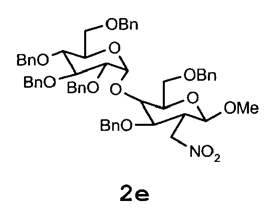 | 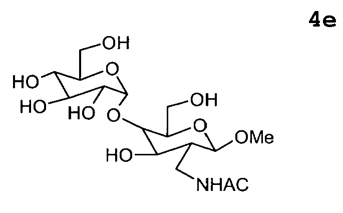 | 71 % |
| 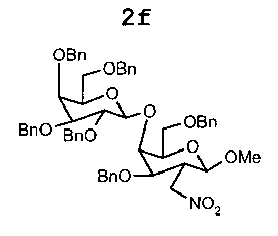 | 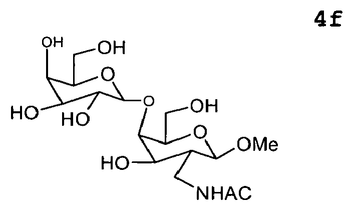 | 73 % |

## Patentansprüche

1. Verfahren zur Herstellung von Glycosamin-Analoga aus natürlichen Kohlenhydraten beziehungsweise Fünfringglycalen oder Sechsringglycalen, **dadurch gekennzeichnet, dass** man in einer ersten Stufe
a) das Glycal durch Addition von Nitroalkan zu Kohlenhydrat 2-C-N-Analoga umsetzt, in denen der Stickstoff als Nitrogruppe vorliegt, und dann in einer zweiten Stufe
b) mittels Reduktion Kohlenhydrat C-N-Analoga als Homologe von Glycosaminen erhält.

2. Verfahren nach Anspruch 1, **dadurch gekennzeichnet, dass** das Nitroalkan 1 bis 4 C-Atome enthält.

3. Verfahren nach Anspruch 1, **dadurch gekennzeichnet, dass** man als Glycal ein Sechsringglycal und als Nitroalkan Nitromethan einsetzt.

4. Verfahren nach Anspruch 1, **dadurch gekennzeichnet, dass** man als Glycal ein Fünfringglycal und als Nitroalkan Nitroethan einsetzt.

5. Verfahren gemäß Anspruch 1, **dadurch gekennzeichnet, dass** die Reduktion in Schritt b) eine selektive Reduktion ist.

6. Verfahren gemäß Anspruch 1, **dadurch gekennzeichnet, dass** die OH-Gruppen des Glycals geschützt sind.

7. Verfahren gemäß Anspruch 1 bis 6, **dadurch gekennzeichnet, dass** die Schutzgruppen der OH-Gruppen im Schritt b) gleichzeitig mit der Bildung des Glycosamins abgespalten werden.

8. Verfahren gemäß Anspruch 1 bis 6, **dadurch gekennzeichnet, dass** die Schutzgruppen der OH-Gruppen im Schritt b) bei der Bildung des Glycosamins erhalten bleiben.
